# EUROPÄISCHE PATENTSCHRIFT

(11) **EP 0 763 346 B1**
(45) Veröffentlichungstag und Bekanntmachung des Hinweises auf die Patenterteilung: **01.09.1999**
(21) Anmeldenummer: 96110542.6
(22) Anmeldetag: 29.06.1996
(51) Int. Cl.: A61B 17/122

(54) **Chirurgischer Clip mit einem Klemmring**
Surgical clip with clamping ring
Clip chirurgical avec anneau de serrage

(30) Priorität: 15.09.1995 DE 19534323
(43) Veröffentlichungstag der Anmeldung: 19.03.1997
(73) Patentinhaber: Aesculap AG & Co. KG, 78532 Tuttlingen (DE)
(72) Erfinder: Back Lothar, D-72514 Inzighofen (DE); Herrmann Gebhard, D-78597 Irndorf (DE); Nesper Markus, D-78532 Tuttlingen (DE); Weisshaupt Dieter, D-78194 Immendingen (DE)
(74) Vertreter: Böhme, Ulrich, Dr. Dipl.-Phys.

(56) Entgegenhaltungen:
- EP-A- 0 346 084
- WO-A-91/08708
- DE-A- 4 319 829

## Beschreibung

Die Erfindung betrifft einen chirurgischen Clip, der zwei elastisch gegeneinander verschwenkbare Arme mit je einem Klemmbacken, einen Endabschnitt, in dem sich die Arme treffen, einen dazwischenliegenden Spannabschnitt und einen Klemmring aufweist, der die Arme umgebend in Längsrichtung verschiebbar ist, wobei ein mit dem Klemmring verbundenes Teil zwischen die Arme eingreift.

Ein derartiger chirurgischer Clip ist beispielsweise aus der DE 43 19 829 C1 bekannt. Es läßt sich mit Hilfe eines speziellen Applikators anlegen und auch wieder lösen. Um den Clip nach dem Anlegen aufspreizen zu können, ist bei dem bekannten Clip vorgesehen, daß der Klemmring einen radial den Klemmring durchsetzenden Stift trägt, der zwischen die Arme eingreift und diese beim Zurückschieben des Klemmrings aufspreizt.

Es handelt sich bei dem Klemmring um ein Teil mit ausserordentlichen kleinen Abmessungen, so daß es sehr schwierig ist, einen solchen Ring mit einem separaten ihn durchsetzenden Stift herzustellen, zumal da der Stift den bekannten Klemmring auch noch auf beiden Seiten außenseitig überragt.

Es ist Aufgabe der Erfindung, einen gattungsgemäßen chirurgischen Clip mit einem Klemmring so zu verbessern, daß der Klemmring in einfacher Weise hergestellt und mit dem Clip schnell und unkompliziert zusammengefügt werden kann.

Diese Aufgabe wird bei einem chirurgischen Clip der gattungsgemäßen Art erfindungsgemäß dadurch gelöst, daß das mit dem Klemmring verbundene zwischen die Arme eingreifende Teil durch diametral gegenüberliegende, einstückig mit dem Klemmring ausgebildete, nach innen in die vom Klemmring umgebene Fläche hineinragende Vorsprünge gebildet wird.

Es wird also nicht mehr ein zwischen die Arme eingreifender Stift verwendet, der mit dem Klemmring verbunden werden muß, sondern der Klemmring selber trägt beidseitig nach innen vorstehende Vorsprünge, die zwischen die Arme eingreifen und diese beim Zurückschieben des Klemmrings aufspreizen.

Dabei können die Vorsprünge als radiale Stege ausgebildet sein, deren Länge vorzugsweise maximal dem halben Radius der vom Klemmring eingeschlossenen Fläche entspricht.

Die Innenkontur der vom Klemmring umschlossenen Fläche angrenzend an die Vorsprünge kann unterschiedlich gewählt werden und wird vorzugsweise an die Außenkontur der Arme des Clips angepaßt. Beispielsweise kann diese Innenkontur ein Kreisbogen oder ein Ellipsenabschnitt sein.

Auch die Außenkontur des Klemmrings kann unterschiedlich gewählt werden, beispielsweise kann der Klemmring kreisförmig oder elliptisch sein.

Bei einer abgewandelten Ausführungsform der Klemmringe ist vorgesehen, daß der Klemmring diametral gegenüberliegende, einstückig mit dem Klemmring ausgebildete, nach außen abstehende Vorsprünge trägt.

Es ist möglich, mittels eines geeigneten Werkzeugs den Ring an diesen nach außen abstehenden Vorsprüngen zu fassen und dadurch quer zur Ebene des Klemmrings zu verschieben, dadurch kann der Clip, auf dem der Klemmring angeordnet ist, von der Schließstellung in die Offenstellung verschoben werden und umgekehrt.

Günstig ist es, wenn die nach innen in den Klemmring hineinragenden Vorsprünge und die nach außen vom Klemmring abstehenden Vorsprünge auf einer Durchmesserlinie des Klemmrings liegen. Es ergibt sich somit ein beidseitiger Angriff an den Klemmring im Bereich der nach innen zwischen die Arme des Gefäßclips eingreifenden Vorsprünge, so daß eine symmetrische Einleitung der Kräfte erfolgt, eine Verklemmung wird dadurch praktisch ausgeschlossen.

Es kann vorgesehen sein, daß die Länge der nach außen abstehenden Vorsprünge maximal dem halben Radius der vom Klemmring eingeschlossenen Fläche entspricht.

Die nach außen abstehenden Vorsprünge können vorzugsweise als radiale Stege ausgebildet sein.

Besonders günstig ist es, wenn die nach innen und/oder die nach außen abstehenden Vorsprünge die gleiche Höhe aufweisen wie der Klemmring und wenn die ebenen Stirnflächen des Klemmrings auch die Vorsprünge begrenzen. Diese ebenen Stirnflächen sind somit auch ebene Seitenflächen der nach innen und/oder außen ragenden Vorsprünge, die somit einen im wesentlichen rechteckigen Querschnitt erhalten.

Bei einer anderen Ausführungsform kann aber auch vorgesehen sein, daß die nach außen abstehenden Vorsprünge eine kreiszylindrische Form aufweisen. Dabei ist es insbesondere günstig, wenn der Durchmesser der kreiszylindrischen, nach außen abstehenden Vorsprünge kleiner ist als die Dicke des Klemmrings.

Die Herstellung eines Klemmrings, der ein sehr kleines Bauteil darstellt, kann gemäß einer bevorzugten Ausführungsform der Erfindung dadurch erfolgen, daß der Klemmring aus einer Scheibe mittels einer Drahterodiermaschine hergestellt wird. Mit dieser ist es möglich, eine Innenausnehmung beliebiger Kontur in eine solche Scheibe einzuarbeiten.

In ähnlicher Weise kann statt einer Drahterodiermaschine auch eine Elektronenstrahleinrichtung oder eine Laserstrahleinrichtung verwendet werden, gegebenenfalls auch eine Wasserstrahleinrichtung. Mit diesen Einrichtungen wird ebenso wie mit der Drahterodiermaschine eine Innenausnehmung mit der entsprechenden Kontur in die Scheibe eingeschnitten.

Bei einer abgewandelten Ausführungsform kann auch vorgesehen sein, daß der Klemmring ein Keramikformkörper ist. Eine solche Ausführung ist insbesondere dann günstig, wenn kreiszylindrische, nach außen abstehende Vorsprünge am Klemmring vorgesehen sind, die nicht ohne weiteres mit einer Drahterodiermaschine oder mit anderen Strahleinrichtungen aus einer Scheibe herausgeschnitten werden.

Die nachfolgende Beschreibung bevorzugter Ausführungsformen der Erfindung dient im Zusammenhang mit der Zeichnung der näheren Erläuterung. Es zeigen:
- Figur 1:: eine schematische Ansicht eines chirurgischen Clips mit einem auf diesem verschiebbaren Klemmring;
- Figur 2:: eine Draufsicht auf einen Klemmring aus Keramik mit nach innen angeformten Vorsprüngen;
- Figur 3:: eine Draufsicht auf einen Klemmring mit nach innen vorstehenden radialen Stegen und dem Draht einer Drahterodiermaschine beim Herstellen der Innenausnehmung des Klemmrings;
- Figur 4:: eine Seitenansicht des Klemmrings der Figur 3 mit dem Draht einer Drahterodiermaschine;
- Figur 5:: eine perspektivische Ansicht eines weiteren bevorzugten Ausführungsbeispiels eines Klemmrings mit nach außen abstehenden Vorsprüngen mit rechteckigem Querschnitt und
- Figur 6:: eine Ansicht ähnlich Figur 5 mit nach außen abstehenden Vorsprüngen mit kreiszylindrischem Querschnitt.

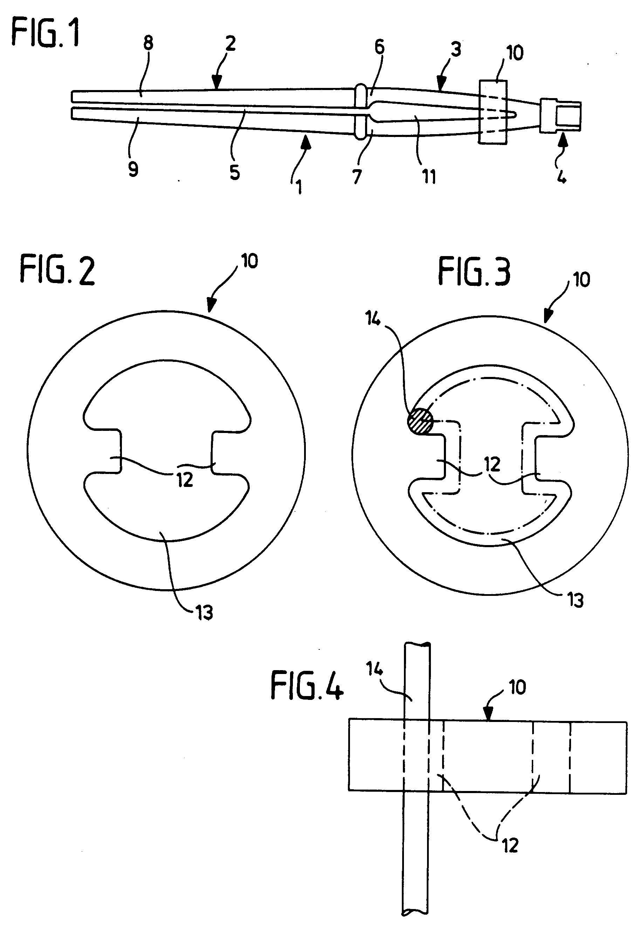

Der in Figur 1 dargestellte chirurgische Clip 1 weist in seiner Längsrichtung hintereinander einen Klemmabschnitt 2, einen Spannabschnitt 3 und einen Endabschnitt 4 auf. Über den größten Teil seiner Länge ist der Clip 1 durch einen Längsschlitz 5 in zwei Arme 6, 7 unterteilt, die sich im Endabschnitt 4 treffen.

Diese beiden Arme 6, 7 bilden im Klemmabschnitt 2 zwei einander gegenüberliegende Klemmbacken 8, 9 aus. Im Spannabschnitt 3 erweitern sich die Arme 6, 7 vom Endabschnitt 4 zu den Klemmbacken 8, 9 hin kegelig, und in diesem Bereich werden die Arme 6, 7 von einem Klemmring 10 umgeben, der auf den Armen 6, 7 im Spannabschnitt 3 in Längsrichtung verschiebbar ist. Beim Vorschieben des Klemmrings 10 drückt er dabei die Arme 6, 7 elastisch zusammen und spannt dadurch die Klemmbacken 8, 9 des Clips 1 gegeneinander.

Der Längsschlitz 7 erweitert sich im Bereich des Spannabschnitts 3 in einen sich zum Endabschnitt 4 hin verjüngenden keilförmigen Zwischenraum 11, und in diesen Zwischenraum 11 greifen Vorsprünge 12 ein, die einstückig mit dem Klemmring 10 ausgebildet sind und nach innen von ihm abstehen (Figuren 2 und 3). Diese Vorsprünge 12 werden vorzugsweise gebildet durch radiale Stege, deren Länge etwa der Hälfte des Radius der vom Klemmring 10 umschlossenen Fläche 13 entspricht.

Die Kontur der umschlossenen Fläche 13 in dem an die Vorsprünge 12 angrenzenden Teil kann kreisbogenförmig sein, es kann aber auch eine andere Form gewählt werden, beispielsweise die Form eines Ellipsenbogens. Insbesondere wird diese Kontur an die Außenkontur der Arme 6, 7 des Clips 1 angepaßt.

Bei dem Ausführungsbeispiel der Figuren 2 und 3 weist der Klemmring nur nach innen gerichtete Vorsprünge 12 auf, an der Außenseite ist der Klemmring kreisförmig ausgebildet.

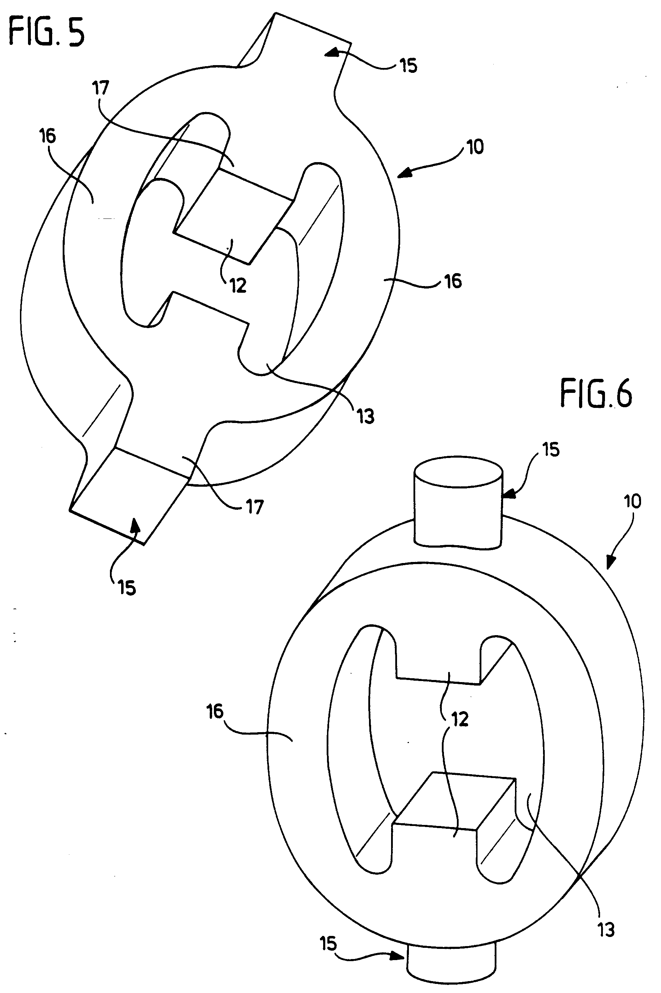

Bei dem in Figur 5 dargestellten abgewandelten Ausführungsbeispiel, das sonst dem Beispiel der Figuren 2 und 3 entspricht und bei dem gleiche Teile daher auch die gleichen Bezugszeichen tragen, sind zusätzlich zwei radial nach außen abstehende Vorsprünge 15 vorgesehen, die den nach innen ragenden Vorsprüngen 12 direkt gegenüberliegen, das heißt durch alle nach innen und nach außen ragenden Vorsprünge 12 beziehungsweise 15 läuft eine gemeinsame Durchmesserlinie des Klemmrings hindurch. Die nach innen ragenden Vorsprünge 12 und die nach außen ragenden Vorsprünge 15 sind im Querschnitt rechteckig ausgebildet, die ebene Stirnfläche 16 auf gegenüberliegenden Seiten des Klemmrings geht in die Seitenflächen 17 dieser Vorsprünge 12 beziehungsweise 15 über, das heißt die Klemmringe sind im gesamten Bereich durch diese ebenen Stirnflächen 16 begrenzt.

Beim Ausführungsbeispiel der Figur 6 hingegen sind die nach außen abstehenden Vorsprünge 15 kreiszylindrisch ausgebildet, wobei der Durchmesser dieser Kreiszylinder kleiner ist als die Dicke des Klemmrings 10.

In beiden Fällen dienen die nach außen abstehenden Vorsprünge 15 als Angriffsflächen für ein Werkzeug, mit dem der Klemmring quer zu seiner Ebene auf dem Clip 1 verschoben werden kann. Dazu ragen die nach außen abstehenden Vorsprünge 15 in entsprechende Ausnehmungen eines Werkzeugs hinein, wie dies an sich bei Klemmringen bekannt ist, die durchgehende Stifte tragen (DE 43 19 829 C1).

Der Klemmring 10 kann beispielsweise als Formteil aus Keramik hergestellt sein, wie dies in Figur 2 dargestellt ist. Diese Herstellungsweise ist insbesondere dann von Vorteil, wenn nach außen abstehende, kreiszylindrische Vorsprünge 15 an den Klemmring angeformt sind, wie dies in Figur 6 dargestellt ist.

Er kann aber auch als Metallteil ausgebildet sein, und dann ist es vorteilhaft, wenn zur Herstellung des Klemmrings 10 von einem scheibenförmigen Körper aus Metall ausgegangen wird, aus dem mittels einer Drahterodiermaschine eine Fläche 13 herausgeschnitten wird, die im wesentlichen kreisförmig ausgebildet ist, wobei die nach innen vorstehenden Vorsprünge 12 in diese Kreisfläche hineinragen. Die tatsächlich ausgeschnittene Fläche ist somit im wesentlichen H-förmig ausgebildet, wie dies aus Figur 3 deutlich wird. Zum Ausschneiden dieser H-förmigen Ausnehmung wird der Draht 14 einer Drahterodiermaschine längs der Kontur dieser ausgeschnittenen Fläche geführt.

## Patentansprüche

1. Chirurgischer Clip (1), der zwei elastisch gegeneinander verschwenkbare Arme (6, 7) mit je einem Klemmbacken (8, 9), einen Endabschnitt (4), in dem sich die Arme (6, 7) treffen, einen dazwischenliegenden Spannabschnitt (3) und einen Klemmring (10) aufweist, auf dem der die Arme (6, 7) umgebende Klemmring (10) in Längsrichtung verschiebbar ist, wobei ein mit dem Klemmring (10) verbundenes Teil (12) zwischen die Arme (6, 7) eingreift, dadurch gekennzeichnet, daß das mit dem Klemmring (10) verbundene zwischen die Arme (6, 7) eingreifende Teil (12) durch diametral gegenüberliegende, einstückig mit dem Klemmring (10) ausgebildete nach innen in die vom Klemmring (10) umgebene Fläche (13) hineinragende Vorsprünge (12) gebildet wird.

2. Chirurgischer Clip nach Anspruch 1, dadurch gekennzeichnet, daß die Vorsprünge (12) als radiale Stege ausgebildet sind.

3. Chirurgischer Clip nach Anspruch 2, dadurch gekennzeichnet, daß die Länge der Vorsprünge (12) maximal dem halben Radius der vom Klemmring (10) eingeschlossenen Fläche (13) entspricht.

4. Chirurgischer Clip nach einem der voranstehenden Ansprüche, dadurch gekennzeichnet, daß die Innenkontur der vom Klemmring (10) umschlossenen Fläche (13) angrenzend an die Vorsprünge (12) ein Kreisbogen ist.

5. Chirurgischer Clip nach einem der Ansprüche 1 bis 3, dadurch gekennzeichnet, daß die Innenkontur der vom Klemmring (10) umschlossenen Fläche (13) angrenzend an die Vorsprünge (12) ein Ellipsenabschnitt ist.

6. Chirurgischer Clip nach einem der voranstehenden Ansprüche, dadurch gekennzeichnet, daß die Außenkontur des Klemmrings (10) kreisförmig ist.

7. Chirurgischer Clip nach einem der Ansprüche 1 bis 5, dadurch gekennzeichnet, daß die Außenkontur des Klemmrings (10) elliptisch ist.

8. Chirurgischer Clip nach einem der voranstehenden Ansprüche, dadurch gekennzeichnet, daß der Klemmring (10) diametral gegenüberliegende, einstückig mit dem Klemmring (10) ausgebildete, nach außen abstehende Vorsprünge (15) trägt.

9. Chirurgischer Clip nach Anspruch 8, dadurch gekennzeichnet, daß die nach innen in den Klemmring (10) hineinragenden Vorsprünge (12) und die nach außen vom Klemmring (10) abstehenden Vorsprünge (15) auf einer Durchmesserlinie des Klemmrings (10) liegen.

10. Chirurgischer Clip nach Anspruch 8 oder 9, dadurch gekennzeichnet, daß die Länge der nach außen abstehenden Vorsprünge (15) maximal dem halben Radius der vom Klemmring (10) eingeschlossenen Fläche (13) entspricht.

11. Chirurgischer Clip nach einem der Ansprüche 8 bis 10, dadurch gekennzeichnet, daß die nach außen abstehenden Vorsprünge (15) als radiale Stege ausgebildet sind.

12. Chirurgischer Clip nach einem der Ansprüche 2 oder 11, dadurch gekennzeichnet, daß die nach innen und/oder die nach außen abstehenden Vorsprünge (12; 15) die gleiche Höhe aufweisen wie der Klemmring (10) und daß die ebenen Stirnflächen (16) des Klemmrings (10) auch die Vorsprünge (12; 15) begrenzen.

13. Chirurgischer Clip nach einem der Ansprüche 8 bis 11, dadurch gekennzeichnet, daß die nach außen abstehenden Vorsprünge (15) eine kreiszylindrische Form aufweisen.

14. Chirurgischer Clip nach Anspruch 13, dadurch gekennzeichnet, daß der Durchmesser der kreiszylindrischen, nach außen abstehenden Vorsprünge (15) kleiner ist als die Dicke des Klemmrings (10).

15. Chirurgischer Clip nach einem der Ansprüche 1 bis 12, dadurch gekennzeichnet, daß er aus einer Scheibe mittels einer Drahterodiermaschine hergestellt ist.

16. Chirurgischer Clip nach einem der Ansprüche 1 bis 12, dadurch gekennzeichnet, daß er aus einer Scheibe mittels einer Elektronenstrahl- oder Laserstrahleinrichtung hergestellt ist.

17. Chirurgischer Clip nach einem der Ansprüche 1 bis 14, dadurch gekennzeichnet, daß er ein Keramikformkörper ist.

## Claims

1. A surgical clip (1) comprising two arms (6, 7) resiliently pivotable towards one another and each provided with a clamping jaw (8, 9), an end portion (4) in which the arms (6, 7) meet, a clamping portion (3) arranged therebetween and a clamping ring (10) surrounding the arms (6, 7) and displaceable on the clamping portion (3) in the longitudinal direction, wherein a part (12), connected to the clamping ring (10), engages between the arms (6, 7), characterised in that the part (12), connected to the clamping ring (10) and engaging between the arms (6, 7), is formed by diametrically opposed projections (12) extending into the area (13) enclosed by the clamping ring (10) and formed in one piece therewith.

2. A surgical clip according to claim 1, characterised in that the projections (12) are formed as radial members.

3. A surgical clip according to claim 2, characterised in that the length of the projections (12) corresponds at most to half the radius of the area (13) enclosed by the clamping ring (10).

4. A surgical clip according to any one of the preceding claims, characterised in that the inner contour of the area (13) enclosed by the clamping ring (10), adjacent to the projections (12), is a circular arc.

5. A surgical clip according to any one of claims 1 to 3,
characterised in that the inner contour of the area (13) enclosed by the clamping ring (10), adjacent to the projections (12), is an elliptical segment.

6. A surgical clip according to any one of the preceding claims, characterised in that the outer contour of the clamping ring (10) is circular.

7. A surgical clip according to any one of claims 1 to 5,
characterised in that the outer contour of the clamping ring (10) is elliptical.

8. A surgical clip according to any one of the preceding claims, characterised in that the clamping ring (10) carries diametrically opposed projections (15) extending outwards and formed in one piece with the clamping ring (10).

9. A surgical clip according to claim 8 characterised in that the projections (12) extending into the clamping ring (10) and the projections (15) extending outwards from the clamping ring (10) are arranged on a diametral line thereof.

10. A surgical clip according to claim 8 or 9, characterised in that the length of the outwardly extending projections (15) corresponds at most to half the radius of the area (13) enclosed by the clamping ring (10).

11. A surgical clip according to any one of claims 8 to 10, characterised in that the outwardly extending projections (15) are formed as radial members.

12. A surgical clip according to either one of claims 2 and 11, characterised in that the inwardly and/or the outwardly extending projections (12, 15) are the same height as the clamping ring (10) and in that the flat end surfaces (16) of the clamping ring (10) also form the boundary of the projections (12, 15).

13. A surgical clip according to any one of claims 8 to 11, characterised in that the outwardly extending projections (15) have a circular cylindrical shape.

14. A surgical clip according to claim 13, characterised in that the diameter of the circular cylindrical, outwardly extending projections (15) is smaller than the thickness of the clamping ring (10).

15. A surgical clip according to any one of claims 1 to 12, characterised in that it is manufactured from a disc by means of a wire spark erosion machine.

16. A surgical clip according to any one of claims 1 to 12, characterised in that it is manufactured from a disc by means of electron-beam or laser-beam apparatus.

17. A surgical clip according to any one of claims 1 to 14, characterised in that it is a ceramic moulding.

## Revendications

1. Pince chirurgicale (1), qui comporte deux branches (6, 7) pouvant pivoter élastiquement l'une par rapport à l'autre comprenant chacune une mâchoire de serrage (8, 9), une section terminale (4) dans laquelle se rencontrent les branches (6, 7), une section de tension (3) située entre les précédentes et un anneau de serrage (10), sur laquelle l'anneau de serrage (10) entourant les branches (6, 7) est déplaçable dans la direction longitudinale, où une partie (12) liée à l'anneau de serrage (10) pénètre entre les branches (6, 7), caractérisée en ce que la partie (12) liée à l'anneau de serrage (10) qui pénètre entre les branches (6, 7) est formée par des protubérances (12) diamétralement opposées, formées d'une pièce avec l'anneau de serrage (10) et pénétrant vers l'intérieur dans la surface (13) entourée par l'anneau de serrage (10).

2. Pince chirurgicale selon la revendication 1, caractérisée en ce que les protubérances (12) sont sous forme de nervures radiales.

3. Pince chirurgicale selon la revendication 2, caractérisée en ce que la longueur des protubérances (12) correspond au maximum au demi rayon de la surface (13) entourée par l'anneau de serrage (10).

4. Pince chirurgicale selon l'une des revendications précédentes, caractérisée en ce que le contour interne de la surface (13) entourée par l'anneau de serrage (10) au voisinage des protubérances (12) est un arc de cercle.

5. Pince chirurgicale selon l'une des revendications 1 à 3, caractérisée en ce que le contour interne de la surface (13) entourée par l'anneau de serrage (10) au voisinage des protubérances (12) est une section d'ellipse.

6. Pince chirurgicale selon l'une des revendications précédentes, caractérisée en ce que le contour externe de l'anneau de serrage (10) est circulaire.

7. Pince chirurgicale selon l'une des revendications 1 à 5, caractérisée en ce que le contour externe de l'anneau de serrage (10) est elliptique.

8. Pince chirurgicale selon l'une des revendications précédentes, caractérisée en ce que l'anneau de serrage (10) porte des protubérances (15) diamétralement opposées, formées d'une pièce avec l'anneau de serrage (10) et qui s'écartent vers l'extérieur.

9. Pince chirurgicale selon la revendication 8, caractérisée en ce que les protubérances (12) qui font saillie vers l'intérieur dans l'anneau de serrage (10) et les protubérances (15) qui s'écartent vers l'extérieur de l'anneau de serrage (10) sont situées sur une ligne diamétrale de l'anneau de serrage (10).

10. Pince chirurgicale selon la revendication 8 ou 9, caractérisée en ce que la longueur des protubérances (15) qui s'écartent vers l'extérieur correspond au maximum au demi rayon de la surface (13) entourée par l'anneau de serrage (10).

11. Pince chirurgicale selon l'une des revendications 8 à 10, caractérisée en ce que les protubérances (15) qui s'écartent vers l'extérieur sont sous forme de nervures radiales.

12. Pince chirurgicale selon l'une des revendications 2 ou 11, caractérisée en ce que les protubérances (12 ; 15) qui font saillie vers l'intérieur et/ou vers l'extérieur présentent la même hauteur que l'anneau de serrage (10) et en ce que les surfaces frontales planes (16) de l'anneau de serrage (10) limitent aussi les protubérances (12 ; 15).

13. Pince chirurgicale selon l'une des revendications 8 à 11, caractérisée en ce que les protubérances (15) qui s'écartent vers l'extérieur présentent une forme cylindrique.

14. Pince chirurgicale selon la revendication 13, caractérisée en ce que le diamètre des protubérances (15) cylindriques qui s'écartent vers l'extérieur est inférieur à l'épaisseur de l'anneau de serrage (10).

15. Pince chirurgicale selon l'une des revendications 1 à 12, caractérisée en ce qu'elle est fabriquée à partir d'une plaque au moyen d'une machine d'étincelage par fil.

16. Pince chirurgicale selon l'une des revendications 1 à 12, caractérisée en ce qu'elle est fabriquée à partir d'une plaque au moyen d'un dispositif à faisceau électronique ou à faisceau laser.

17. Pince chirurgicale selon l'une des revendications 1 à 14, caractérisée en ce qu'il s'agit d'un corps moulé en céramique.
